# EUROPEAN PATENT SPECIFICATION

(11) **EP 1 395 681 B1**
(45) Date of publication and mention of the grant of the patent: **26.07.2006**
(21) Application number: 02729732.4
(22) Date of filing: 29.05.2002
(51) Int. Cl.: C12Q 1/68

(54) **PHARMACOLOGICAL APPLICATIONS OF MITOCHONDRIAL DNA ASSAYS**
PHARMAKOLOGISCHE ANWENDUNGEN VON MITOCHONDRIELLEN DNA ASSAYS
APPLICATIONS PHARMACOLOGIQUES D'ANALYSES D'ADN MITOCHONDRIAL

(30) Priority: 29.05.2001 US 293523 P
(43) Date of publication of application: 10.03.2004
(73) Proprietor: THE UNIVERSITY OF BRITISH COLUMBIA, Vancouver, British Columbia V6T 1Z3 (CA)
(72) Inventor: COTE, Helene, Vancouver, British Columbia V6S 1H9 (CA); MONTANER, Julio, Vancouver, British Columbia V6N 2L9 (CA); O'SHAUGHNESSY, Michael, V., Maple Rigde, British Columbia V2W 1P5 (CA)
(74) Representative: De Clercq, Ann G. Y.
(86) International application number: PCT/CA2002/000796
(87) International publication number: WO 2002/097124

(56) References cited:
- WO-A-00/50043
- WO-A-01/35096
- ARNAUDO E ET AL: "Depletion of muscle mitochondrial DNA in AIDS patients with zidovudine-induced myopathy." LANCET. ENGLAND 2 MAR 1991, vol. 337, no. 8740, 2 March 1991 (1991-03-02), pages 508-510, XP001074366 ISSN: 0140-6736
- CHURCH JOSEPH A ET AL: "Mitochondrial DNA depletion, near-fatal metabolic acidosis, and liver failure in an HIV-infected child treated with combination antiretroviral therapy." JOURNAL OF PEDIATRICS, vol. 138, no. 5, May 2001 (2001-05), pages 748-751, XP001100095 ISSN: 0022-3476
- LEWIS WILLIAM ET AL: "Depletion of mitochondrial DNA, destruction of mitochondria, and accumulation of lipid droplets results from fialuridine treatment in woodchucks (Marmota monax)." LABORATORY INVESTIGATION, vol. 76, no. 1, 1997, pages 77-87, XP002213602 ISSN: 0023-6837
- BRINKMAN K ET AL: "Mitochondrial toxicity induced by nucleoside-analogue reverse-transcriptase inhibitors is a key factor in the pathogenesis of antiretroviral-therapy-related lipodystrophy" LANCET, XX, XX, vol. 354, no. 9184, 25 September 1999 (1999-09-25), pages 1112-1115, XP004262656 ISSN: 0140-6736
- KAKUDA THOMAS N: "Pharmacology of nucleoside and nucleotide reverse transcriptase inhibitor-induced mitochondrial toxicity." CLINICAL THERAPEUTICS, vol. 22, no. 6, June 2000 (2000-06), pages 685-708, XP002213603 ISSN: 0149-2918
- MEDINA DANIEL J ET AL: "Comparison of mitochondrial morphology, mitochondrial DNA content, and cell viability in cultured cells treated with three anti-human immunodeficiency virus dideoxynucleosides." ANTIMICROBIAL AGENTS AND CHEMOTHERAPY, vol. 38, no. 8, 1994, pages 1824-1828, XP002213604 ISSN: 0066-4804
- BERLIN KRISTEN R SCHNEIDER ET AL: "Dequalinium induces a selective depletion of mitochondrial DNA from HeLa human cervical carcinoma cells." EXPERIMENTAL CELL RESEARCH, vol. 245, no. 1, 25 November 1998 (1998-11-25), pages 137-145, XP002213605 ISSN: 0014-4827
- KAO S H ET AL: "Multiple deletions of mitochondrial DNA are associated with the decline of motility and fertility of human spermatozoa." MOLECULAR HUMAN REPRODUCTION. ENGLAND JUL 1998, vol. 4, no. 7, July 1998 (1998-07), pages 657-666, XP008008279 ISSN: 1360-9947

## Description

### FIELD OF THE INVENTION

The invention is in the field of diagnostics and therapeutics involving nucleic acids.

### BACKGROUND OF THE INVENTION

Nucleoside analogue reverse transcriptase inhibitors (NRTIs) represent the cornerstone of antiretroviral therapy in HIV infection. Through their incorporation into elongating viral DNA molecules transcribed by the HIV reverse transcriptase, they effectively inhibit viral replication. However, NRTIs can also inhibit the human DNA polymerase gamma (POLγ) (Martin *et al.,* 1994) and thereby mitochondrial DNA (mtDNA) replication, leading to mtDNA depletion and drug toxicity (Brinkman et al., 1998; Lewis and Dalakas, 1995; Kakuda, 2000). This mitochondrial toxicity (MT) leads to a number of adverse effects including lactic acidosis, myopathy, cardiomyopathy, neuropathy, liver steatosis, nephrotic toxicity and pancreatitis (Lewis and Dalakas, 1995; others). The wide variety of clinical symptoms caused by NRTIs is reminiscent of the complex array of symptoms produced by diseases resulting from mtDNA mutations (for review see Wallace, 1999).

Early studies on zidovudine-induced myopathy have shown a decrease in total mtDNA isolated from muscle biopsies in both humans (Arnaudo et al., 1991) and rats (Lewis et al., 1992). In vitro studies with various anti-HIV nucleoside analogues have also shown that NRTIs cause a reduction in the mitochondrial content of human lymphoblastoid cells (Chen et al., 1991; Zhang et al., 1994), CEM cells (Medina et al., 1994) and HepG2 cells (Pan-Zhou et al., 2000). Recently, large hepatic mtDNA deletions but no mtDNA depletion were reported in association with a fatal case of lactic acidosis during antiretroviral therapy (Bartley et al., 2001). It has been suggested that mtDNA depletion (or deletion) may cause a decrease in mitochondrial RNA, mtDNA-encoded protein synthesis and ultimately mitochondrial dysfunction (Lewis et al., 1992). At the cellular level, the consequences of such toxicity are decreased oxidative phosphorylation, intracellular lipid accumulation and lactic acid accumulation. At the physiological level, this may translate into hyperlactatemia that may or may not be accompanied by other mitochondrial toxicity symptoms such as fatigue, rapid weight loss, lipid abnormalities, and liver steatosis. Chronic hyperlactatemia is likely a reflection of impaired hepatic lactate clearance (Brinkman, 2000) which may or may not find its etiology in the nucleoside analogue toxicity itself. Considering the long term nature of antiretroviral therapy, this recently identified syndrome of hyperlactatemia appears to be seen with increasing frequency in HIV infected patients on antiretroviral therapy (Lonergan et al., 2000; Gerard et al., 2000). Its presentation, severity and frequency are distinct from those of acute lactic acidosis, a rare NRTI adverse effect which is often fatal (Fortgang et al., 1995; Megarbane et al., 2000). However, whether hyperlactatemia is a risk factor for lactic acidosis remains unclear.

The diagnosis and treatment of patients with this NRTI-induced hyperlactatemia remains problematic. For example, it can be challenging to diagnose the condition because the early toxicity symptoms of fatigue and wasting are relatively common in AIDS patients and can resemble disease progression. Once mitochondrial toxicity is recognized, treatment may consist of terminating NRTI therapy and monitoring improvement in the patient condition and blood lactic acid levels (Brinkman, 2000; Moyle, 2000). Diagnosis of mitochondrial dysfunction may be made by muscle or liver biopsy, but this may not be practical for routine screening and monitoring. A random venous lactic acid (RVLA) measurement is a useful marker but its reliability is limited by its sensitivity to external factors that are difficult to control. The monitoring of RVLA in a cohort of antiretroviral-treated HIV positive patients has demonstrated that consecutive RVLA measurements were consistent within individuals and were frequently above the normal range (Harris et al., 2000). Moreover, a significant conelation has been found between abnormal RVLA and treatment with stavudine (D4T) and hydroxyurea, as well as length of time on D4T (Harris et al., 2000). However, elevated .RVLA levels are not specific to nucleoside-related mitochondrial toxicity and can have other causes such as infection. There is little *in vivo* data available for nucleosides-related toxicities observed with NRTIs other than zidovudine.

Arnaudo, E and Dalakas, M, (1991), describe destructive mitochondrial myopathy with histological features of ragged-red fibers (RRF) and proliferation of abnormal mitochondria in patients with human immunodeficiency virus (HIV) infection treated long-term with zidovudine therapy and detected reduced amounts of mitochondrial DNA in muscle biopsy specimens by means of Southern blotting.

Church et al. (2001) describe abnormal mitochondrial morphology, reduced mitochondrial enzyme activities, and mitochondrial DNA depletion in muscle biopsies of a child with controlled human immunodeficiency virus infection presented with neurologic deterioration, lactic acid acidosis, and organic aciduria.

WO01/35096 describes compositions and methods for treating diseases associated with altered mitochondrial function, and in particular, for type 2 diabetes mellitus.

### SUMMARY OF THE INVENTION

In one aspect, the invention provides a method for monitoring toxicity of a drug treatment, comprising measuring the relative mitochondrial DNA content of cells in a subject undergoing treatment with the drug. The mitochondrial DNA content may be measured relative to the amount of nuclear DNA in the cells of the subject. The amount of DNA may for example be measured by a polymerase chain reaction, such as a quantitative polymerase chain reaction, wherein amplification of the mitochondrial DNA is compared to amplification of a reference DNA. The methods of the invention may be used on human patients suffering from a disease, such as HIV infection, such as patients undergoing treatment with a nucleoside analogue (such as D4T). In alternative aspects, methods of the invention may be used to monitor the mitochondrial toxicity of test compounds in animal models, where for example the animal model subject is undergoing treatment with a drug. The assay may for example be conducted on cells extracted from a tissue, such as cells obtained from organ biopsies (which may for example be obtained post-mortem).

In one aspect, for example, the present invention discloses that mtDNA from peripheral blood mononuclear cells (PBMCs) is depleted in patients who are experiencing nucleoside-related mitochondrial toxicity (MT) symptoms. A semi-quantitative assay is accordingly provided to detect and monitor NRTI-related mitochondrial toxicity from a venous blood sample. In alternative embodiments, the methods of the invention may comprise the step of discontinuing treatment of the subject with a nucleotide analogue, such as D4T, when the relative mitochondrial DNA content of the cells falls below a predetermined level, such as when the predetermined level of mitochondrial DNA is 5,10, 15, 20, 25, 30 or 35% of a baseline level of mitochondrial DNA, wherein the baseline level of mitochondrial DNA is measured before the subject is treated with the drug, or is measured in a control subject.

In particular, the present invention relates to an in vitro method for monitoring toxicity of a nucleic acid precursor drug treatment of a subject suffering from an HIV infection, comprising measuring the mitochondrial DNA content relative to the amount of nuclear DNA in cells in a peripheral blood sample from a subject undergoing treatment with the drug. In a further embodiment, the method of the invention further comprises the step of determining whether the ratio of mitochondrial DNA to nuclear DNA falls below a predetermined level of 0.5 of a baseline ratio, wherein the baseline ratio is measured before the subject is treated with the drug, or is measured in a control subject. According to the invention the predetermined level may be 0.45, 0.40, 0.35 or 0.30 of the baseline ratio.

In the method of the present invention, the amount of DNA may be measured by a polymerase chain reaction. Said polymerase chain reaction may be a quantitative polymerase chain reaction, wherein amplification of the mitochondrial DNA is compared to amplification of a reference DNA. The polymerase chain reaction may also be a real-time polymerase chain reaction wherein an amplification product is detected with a hybridization probe.

According to the present invention, the subject may be human or a non-human animal, wherein the human subject may be suffering from HIV infection.

Furthermore, according to the present invention the drug may be a nucleoside or nucleotide analogue, wherein the nucleoside analogue may be selected from the group consisting of AZT, ddI, ddC, d4T, 3Tc, Abacavir and D4T and wherein the nucleotide analogue may be selected from the group consisting of Tenofovir and Cidofovir. Furthermore, according to the present invention the drug may be a reverse transcriptase inhibitor.

In the method of the present inventon, the consequence of said toxicity may be chosen from the group consisting of myopathy, cardiomyopathy, neuropathy, liver steatosis, nephrotic toxicity, pancreatitis, decreased oxidative phosphorylation, lactic acidosis, lipid abnormalities, hyperlactatemia, low anaerobic threshold in cardiopulmonary testing, fatigue and rapid weight loss.

### BRIEF DESCRIPTION OF THE DRAWINGS

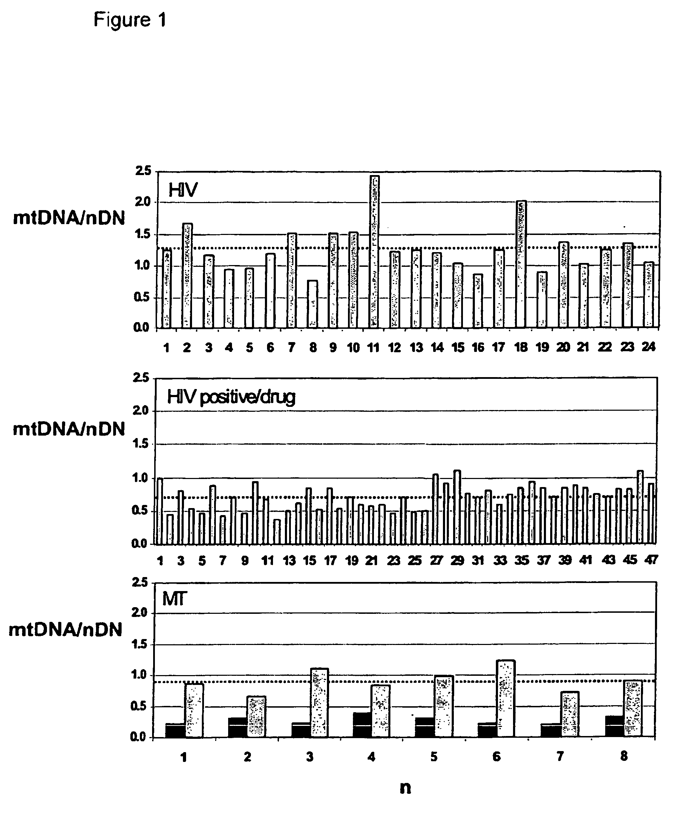

**Figure 1:** PBMC mtDNA/DDNA ratios of A) HIV negative males, B) HIV positive/drug naive males (no PI/NNRT1 were detectable in plasma samples), C) HIV positive/symptomatic MT patients. The black bar represents the lowest mtDNA/nDNA ratio measured during antiretroviral therapy and the gray bar represents the highest ratio reached after interrupting the initial antiretroviral therapy.

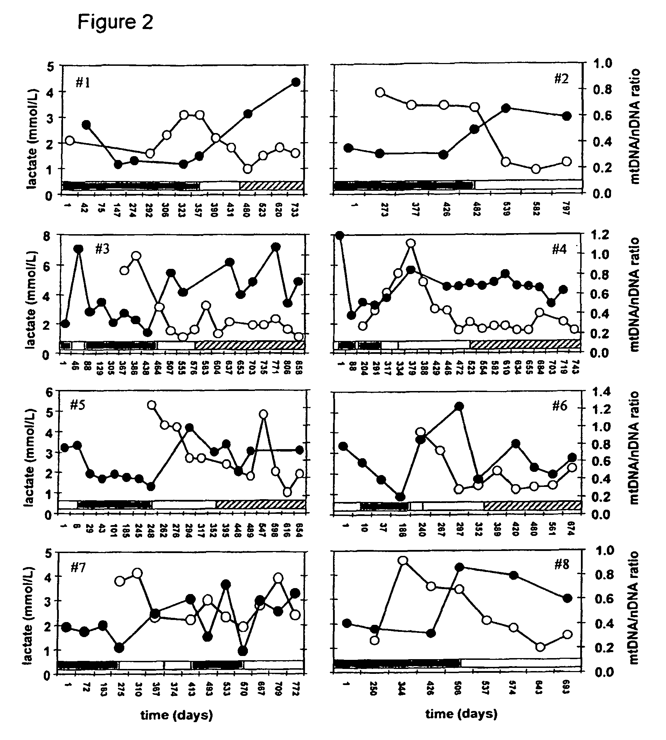

**Figure 2:** Longitudinal analysis of venous lactate levels (left axis) and mtDNA levels (right axis) and antiretroviral drug regimen (bottom bar) over time, for the patients with MT symptoms. The bar is colored dark gray when the patients were on the drug regimen that led to MT, white when off all antiretroviral drugs, and hatched when receiving a new regimen that does not include D4T (see Table 1). This antiretroviral drug data is based on the medical chart information, drug prescription dates and plasma drug levels. Pale gray regions indicate samples in which the plasma drug levels for PI and/or NNRTI were measured at >2 standard deviations below the average through concentration (according to the drug manufacturer's monograph). Note that for clarity and simplicity time is expressed as the distinct days on which the samples were collected.

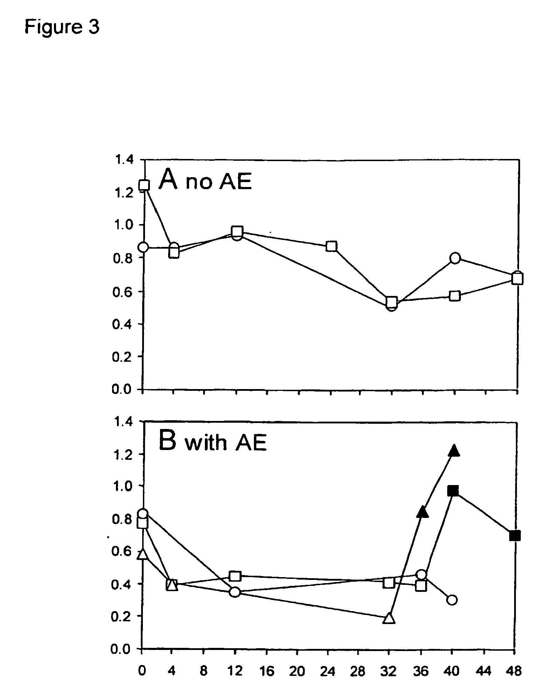

**Figure 3:** Longitudinal analysis of mtDNA levels (left axis, expressed as the ratio mtDNA/nDNA) for patients receiving the antiretroviral regimen stawdine (d4T), didanosine (ddl) and efavirenz (EFV) over a time course shown in days (bottom axis). A) Two patients who did not have adverse effects. B) Three patients who did have adverse effects (hyperlactatemia, weight loss, +/- peripheral neuropathy). Open symbols = on therapy, close symbols = off therapy because of adverse side effects. Patients represented by squares also received hydroxyurea.

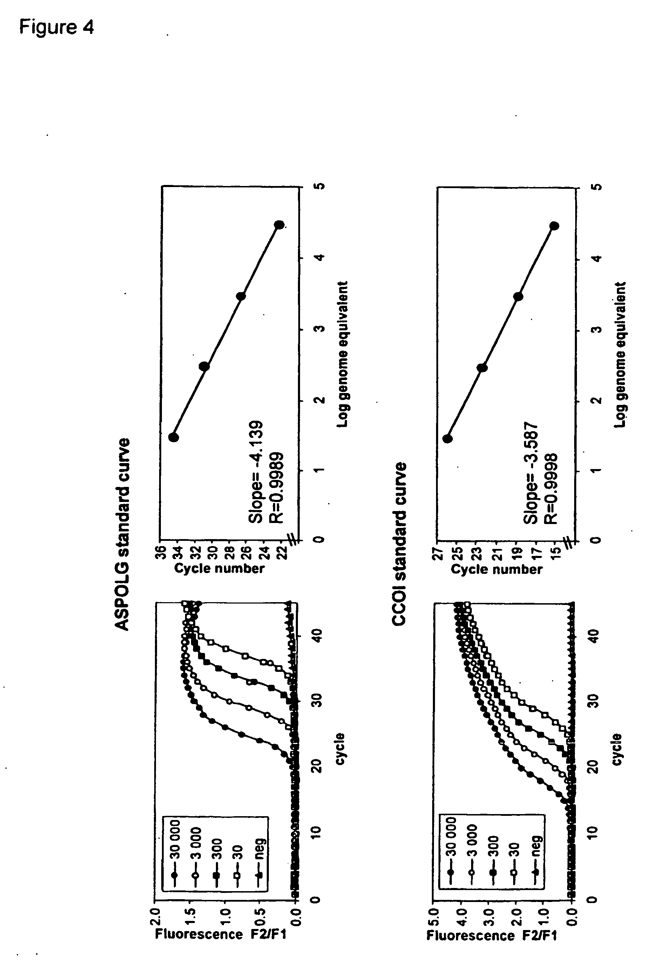

**Figure 4:** Typical LightCycler Real-Time PCR standard curves generated for the nuclear gene ASPOLG and the mitochondrial gene CCOI, using serial dilutions of the pooled DNA extracts from HIV negative male volunteers. The numbers (30 to 30,000) shown in the standard curve for the nuclear gene indicate the number of nuclear-genome equivalents included in each run. The same numbers were assigned in the standard curve for the mitochondrial gene (although they do not represent a calculated copy number of the mitochondrial gene). The nuclear-genome-equivalent content of the HIV negative DNA pool was determined by calibration with a control human DNA of known nuclear-genome-equivalent concentration (as for example may be available from Roche Applied Science, Laval, Quebec, Canada).

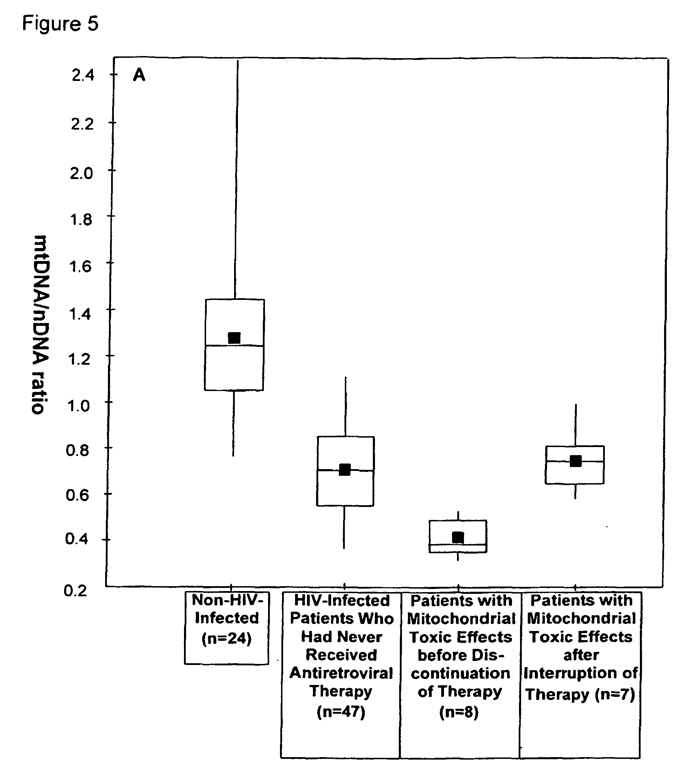

**Figure 5:** Comparative box plots of mtDNA/nDNA ratios between HIV uninfected males (mean ± SD=1.28 ± 0.38, N=24), HIV infected asymptomatic/antiretroviral naive males (no detectable PI/NNRTI in plasma samples) (0.72 ± 0.19, N=47), and HIV infected/antiretroviral treated symptomatic mitochondrial toxicity patients. For the latter, the -on therapy- (0.41 ± 0.08, N=8) and -off therapy- (0.74 ± 0.13, N=7) mtDNA/nDNA ratios are depicted. The lines indicate the maximum and minimum mtDNA/nDNA ratios observed within each group, the edges of the box indicate the 25% and 75% quartiles, the middle line indicates the median and the black square shows the mean mtDNA/nDNA ratio.

### DETAILED DESCRIPTION OF THE INVENTION

In one aspect, the invention provides an assay to quantify mitochondrial DNA (mtDNA) in peripheral blood cells and thereby determine whether the mtDNA levels are at levels indicative of mitochondrial deficit, such as may be caused by toxicity of a therapeutic treatment. The invention provides assays to determine the relative amount of mitochondrial DNA in a subject, such as a subject undergoing drug treatment. The subject may for example be a human patient undergoing treatment for an HIV infection with a nucleic acid precursor such as a nucleoside or nucleotide analogue. The assays of the invention may include PCR assays, such semi-quantitative or quantitative PCR involving the co-amplification of a mitochondrial sequence and a reference sequence, such as a genomic sequence. Information from such assays may be evaluated to provide a ratio of mithchondrial DNA to nuclear DNA in the cells of the subject.

For example, such assays may be carried out in HIV patients on antiretroviral therapy. In one aspect of the invention, antiretroviral therapies may therefore be modulated in accordance with the results of the mtDNA assays of the invention. In alternative aspects of the invention, sample may be tested from patients undergoing therapy with nucleic acid precursors, such as nucleoside or nucleotide analogues. Nucleoside analogs may for example include AZT and ZDV (Retrovir), ddI (Videx and Videx EC) ddC (Hivid) d4T (Zerit) 3TC (Epivir) ABC (Ziagen). Nucleoside analogues are any modified versions of a natural nucleoside. Nucleoside analogs may take the place of the natural nucleosides, blocking the completion of a viral DNA chain during infection of a new cell by HIV. Alternative nucleic acid precursors include nucleotide analogues, such as Cidofovir (also known as HPMPC). Nucleoside analogs and other nucleic acid precursors may also be used in cancer chemotherapy, to inhibit replication of cancer cells.

Samples from patients that are HIV positive may be tested in various aspects of the invention, including such patients who are undergoing nucleoside analogue therapy. Similarly, cancer patients may be monitored with diagnostic assays of the invention, including cancer patients undergoing therapy with a nucleic acid precursor.

In the examples illustrating various aspects of the invention, mitochondrial DNA is shown to be depleted in peripheral blood cells from patients undergoing therapy with a nucleic acid precursor who are suffering from antiretroviral drug-related hyperlactatemia and other mitochondrial toxicity symptoms such as fatigue and rapid weight loss. This depletion preceded a rise in venous lactic acid levels, an observation that is consistent with hyperlactatemia being a consequence of mtDNA depletion. In some embodiments, assays of the invention could therefore provide clinical information before mitochondrial toxicity develops or becomes severe enough to be accompanied by hyperlactatemia. In some cases, even short periods of time with reduced plasma drug concentration are shown to result in increased mtDNA/nDNA ratios, showing that the tests of the invention may advantageously be performed while patients are actively undergoing therapy.

The depletion in mtDNA levels is shown to be reversible in some patients, as shown by the examples herein which illustrate a rise in mtDNA/nDNA ratios following antiretroviral therapy interruption. In the relevant examples, this was accompanied by a gradual return to normal VLA levels. It is shown in the examples that mtDNA levels were significantly lower in the HIV positive control group than in the HIV negative one, a difference that was not explained by the lower CD4 counts in the former. Accordingly, in one aspect the invention provides a diagnostic test that may provide information indicative of HIV infection. It was also found that within the HIV positive control group, storage prior to DNA extraction showed no significant correlation with the mtDNA/nDNA ratios measured (data not shown). In some cases, it was found that severe symptoms may occur when the mtDNA levels fall below approximately 30% to 20% of normal. Alternative measures of mtDNA depletion that may suggest clinical intervention are mtDNA to nDNA ratios of less than 0.5, 0.45, 0.4, 0.35 or 0.3.

In alternative embodiments, drug treatment may be discontinued when the ratio of mtDNA to nDNA, as determined herein, falls below a threshold value such as 0.5, 0.45, 0.4, 0.35 or 0.3 as measured with respect to a control sample. Methods of the invention may comprise treating the patient with an alternative nucleoside analogue after discontinuing treatment of the subject with a particular nucleoside such as D4T. Alternatively, such patients may be treated with mitochondrial therapeutics, i.e. compositions of benefit to mitochondria, such as mitochondrial enzyme co-factors or precursors. In some embodiments, such mitochondrial therapeutics may for example be selected from the group consisting of riboflavin (vitamin B2), coenzyme Q10, vitamin B1 (thiamine), vitamin B 12, vitamin K, 1-acetyl carnitine, N-acetyl cysteine and nicotinamide.

In alternative embodiments, the rate of change of mtDNA concentration over a time period may be determined to provide additional diagnostic information. For some patients, a relatively rapid decrease in the relative amount of mtDNA may be indicative of drug toxicity or a disease state. For example, as shown in Figure 3B, a relatively rapid decrease of on the order of 50% or more (or more than 40% in some cases) in the relative amount ofmtDNA compared to nDNA over a period of less than eight to ten days may be indicative that a patient will eventually have adverse effects from a drug , and may therefore need to be monitored more closely, and may eventually need to be rotated to alternative treatment or have drug treatment discontinued.

As was the case for other studies of hyperlactatemia (Lonergan et al., 2000; Gerard et al., 2000; John et al., 2001), all the patients were receiving D4T as part of their drug regimen at the time the toxicity developed. It was surprisingly discovered that the mtDNA/nDNA ratios measured while off antiretroviral therapy were very similar to those observed once patients resumed nucleoside-containing therapy that excluded D4T. (Table 1, Figure 2 patients 3, 4, 5). Accordingly, one aspect of the invention provides for discontinuance of a drug therapy when a threshold depletion of mtDNA is detected, in conjunction with a switch to an alternative drug. For example, patients may switch from one nucleoside analogue regimen to an alternative nucleoside analogue regimen.

The frequency of lactic acidosis has been estimated in a 1995 retrospective study to lie between one and two cases per 1000 person-year treated with NRTIs (Fortgang et al., 1995). However, another study with a broadened case definition of hyperlactatemia accompanied by either abdominal symptoms or unaccounted for elevated alanine transferase estimated the incidence at 20.9 cases per 1000 person-years of treatment with NRTIs (Lonergan et al., 2000). A large proportion of patients receiving antiretroviral therapy in our cohort exhibit mild to moderate chronic hyperlactatemia, most of them asymptomatic. The assay of the invention may be used to monitor and evaluate the clinical consequences of mitochondrial toxicity and chronic hyperlactatemia in such patients.

In one aspect of the invention, protocols are provided that avoid the necessity to determine mtDNA copy number per se, facilitating instead a determination of the relative amount of mtDNA, such as the amount relative to a nDNA sequence. In some aspects, this approach may simplify the diagnostic assays of the invention. For example, as shown in Figure 4, numbers (30 to 30,000) representing nuclear-genome-equivalents are assigned to nDNA amplification standards, as determined by calibration with a control human DNA of known nuclear-genome-equivalent concentration. The same numbers are arbitrarily assigned to the corresponding standard curves for the mitochondrial gene (although they do not represent a calculated copy number of the mitochondrial gene). In an alternative approach, the numbers representing nuclear-genome-equivalents may be arbitrarily assigned to the nDNA amplification standards, based only on the degree of sample dilution (so that the number reflect the relative copy number of nuclear-genome-equivalents, but not the absolute value of such equivalents), and these arbitrary numbers may similarly be assigned to the mtDNA amplification standards. The results of the assays of the invention may then be expressed by the ratio of mtDNA to nDNA, without the need to determine absolute mtDNA copy numbers. In such embodiments, it may be preferable to utilize an initial concentration of sample DNA that provides sufficient PCR template so that the number of amplification cycles is within the range which provides the most reliable results, such as from a minimum of any integer from 5 to 15 up to a maximum of any integer from 15 to 40.

A process for comparing the relative abundance of NA sequences, comprising:
a) measuring the amplification kinetics of a nuclear NA sequence under a nuclear amplification reaction condition in a first nuclear control sample and in a second nuclear control sample, to obtain control nuclear amplification measurements, wherein the first and the second nuclear control samples have different concentrations of the nuclear NA sequence;
b) constructing a control nuclear NA sequence dataset from the control nuclear amplification measurments, to obtain a model standard relationship between amplification kinetics and concentration for the nuclear NA sequence;
c) measuring the amplification kinetics of a mitochondrial NA sequence under a mitochondrial amplification reaction condition in a first mitochondrial control sample and in a second mitochondrial control sample, to obtain control mitochondrial amplification measurements, wherein the first and the second mitochondrial control samples have different concentrations of the mitochondrial NA sequence;
d) constructing a control mitochondrial NA sequence dataset from the control mitochondrial amplification measurments, to obtain a model standard relationship between amplification kinetics and concentration for the mitochondrial NA sequence;
e) measuring the amplification kinetics of the nuclear NA sequence under the nuclear amplification reaction conditions in a test sample, to obtain a test sample nuclear amplification measurement;
f) applying the model standard relationship between amplification kinetics and concentration for the nuclear NA sequence to the test sample nuclear amplification measurement, to obtain a test sample nuclear NA sequence concentration measurement;
g) measure the amplification kinetics of the mitochondrial NA sequence under the mitochondrial amplification reaction conditions in the test sample, to obtain a test sample mitochondrial amplification measurement;
h) applying the model standard relationship between amplification kinetics and concentration for the mitochondrial NA sequence to the test sample mitochondrial amplification measurement, to obtain a test sample mitochondrial NA sequence concentration measurement;
i) comparing the test sample nuclear NA sequence concentration measurement to the test sample mitochondrial NA sequence concentration measurement, to determine the relative concentration of the mitochondrial NA sequence compared to the nuclear NA sequence in the test sample.

In alternative aspects of the invention, cells for use in assays of the invention may be obtained, for example by biopsy, from a variety of tissues, such as from heart, brain, kidney, fat or liver.

In alternative embodiments, the diagnostic tests of the invention may be used for the diagnosis of a disease condition. For example, measuring the relative amount of a mitochondrial DNA in cells in a sample from a subject, such as a sample of peripheral blood, may provide information relating to diseases or symptoms such as male infertility, organ failure, hepatitis A, B or C infection, HIV infection, arthritis, a neurological disease (including but not limited to Alzheimer's, Parkinson's, Huntingtin's). The diagnosis of such conditions may for example be undertaken when the conditions are treated with a drug, such as a nucleic acid precursor, or the conditions are caused by such a drug.

In alternative aspects, the invention provides kits having components for use in methods of the invention. Such kits may comprise PCR components, as set out in detail below, including PCR primers specific for a mtDNA sequence and for a nDNA sequence. Such kits may also include written instructions for carrying out the methods of the invention as described herein.

In alternative embodiments, a variety of techniques may be used to measure the relative amount of a mitochondrial DNA in cells. Methods of quantitative PCR are for example disclosed in the following documents, United States Patent No. 6,180,349 issued to Ginzinger, *et al.* January 30, 2001; United States Patent No. 6,033,854 issued to Kurnit, *et al.* March 7, 2000; and United States Patent No. 5,972,602 issued to Hyland, *et al.* October 26, 1999.

### Example 1

As illustrated in the following examples, nucleoside-related mitochondrial toxicity is associated with a significant decrease in blood cell mtDNA content, an effect that is reversible upon therapy interruption. An assay is provided to monitor mitochondrial toxicity, for example in patients on antiretroviral therapy. Methods of the invention may be adapted to assess the toxicity of other drugs and to monitor the mitochondrial health of patients with inherited diseases that affect mtDNA levels.

### Materials and Methods

Longitudinal blood samples were studied retrospectively from 8 patients whose antiretroviral therapy was interrupted because of mitochondrial toxicity symptoms. Their symptoms included moderate hyperlactatemia, fatigue, rapid weight loss and low anaerobic threshold in cardiopulmonary testing. Total DNA was extracted from blood cells and both a nuclear gene and a mitochondrial gene were amplified and quantified by Real-Time PCR using hybridization probes. The mtDNA levels were expressed as a ratio of the mitochondrial over nuclear DNA (mtDNA/nDNA).

### Sample collection and DNA extraction

Buffycoats were collected from the same blood samples used for plasma viral load determination and stored frozen at -70°C until used. Plasma viral loads were measured using the Amplicor Ultra-Sensitive HIV-1 Monitor assay (Roche Molecular Diagnostic Systems, Branchburg, New Jersey). Total DNA was extracted from 200 µL of buffycoat using the QlAamp DNA Blood Mini kit (QIAGEN, Missisauga, Ontario, Canada) according to the manufacturer's protocol, and resuspended in 200 µL of elution buffer. For the standard curves, similar samples were collected from 24 HIV negative male volunteers and the DNA was extracted and pooled. The nuclear genome equivalent (g.eq.) content of the HIV negative DNA pool was determined by calibration with control kit human DNA of known nuclear g.eq. concentration (Roche Molecular Biochemicals, Laval, Quebec, Canada).

### Random venous lactic acid measurement

Venous specimens for lactic acid determination were collected in sodium fluoride/potassium oxalate tubes, with normal tourniquet and no specific patient instruction other than the avoidance of fist clenching or hand pumping. The laboratory reference range is 0.7 to 2.1 mmol/L.

### Quantitative real-time PCR

For the mtDNA CCOI gene, the CCOHF 5'-TTCGCCGACCGTTGACTATT-3' and CCOI2R 5'-AAGATTATTACAAATGCATGGGC-3' primers were used for the PCR amplification and the oligonucleotides 3'-Fluorescein-labeled CCOIPR1 5'-GCCAGCCAGGCAACCTTCTAGG-F-3' and 5'LC Red640-labeled CCOIPR2 5'-L-AACGACCACATCTACAACGTTATCGTCAC-P-3', the 3' end of the latter blocked with a phosphate molecule, were used as hybridization probes. For the nDNA ASPOLγ gene, the ASPG3F 5'-GAGCTGTTGACGGAAAGGAG-3' and ASPG4R 5'-CAGAAGAGAATCCCGGCTAAG-3' primers were used for the PCR and the oligonucleotides 3'-Fluorescein-labeled ASPGPR1 5'-GAGGCGCTGTTAGAGATCTGTCAGAGA-F-3' and 5'LC Red640-labeled, 3'-Phosphate-blocked ASPGPR2 5'-L-GGCATTTCCTAAGTGGAAGCAAGCA-P-3' were used as hybridization probes. The real-time PCR reactions were done separately and in duplicate for each gene, using the LightCycler FastStart DNA Master Hybridization Probes kit (Roche Molecular Biochemicals, Laval, Quebec, Canada). The PCR reactions contained 5 mM MgCl₂, 0.5 µM of each primer, 0.1 µM 3'-Fluorescein probe, 0.2 µM 5'LC Red640 probe and 4 uL of a 1:10 dilution of the DNA extract in elution buffer. The PCR amplification consisted of a single denaturation/enzyme activation step of 10 min at 95°C followed by 45 cycles of 0 s/95°C, 10 s/60°C, 5 s/72°C, with a 20°C/s temperature transition rate. The gain settings were F1=1, F2=8 and a single fluorescence acquisition was made at the end of each annealing step. An external standard curve of 30, 300, 3000, and 30000 nuclear g.eq. was included in each LightCycler run, and the same nuclear g. eq values were used for both the nuclear (ASPOLγ) and the mitochondrial (CCOI) genes. The data were analyzed using the second derivative maximum of each amplification reaction and relating it to its respective standard curve. Results from the quantitative PCR were expressed as the relative ratio of the mean mtDNA g.eq. of duplicate measurements over the mean nDNA g.eq. of duplicate measurements for a given extract (mtDNA/nDNA), a ratio arbitrarily set around 1.0 by the fact that the same nuclear g. eq. values were used to generate both standard curves.

In some embodiments, PCR methods of the invention may be real-time polymerase chain reactions wherein an amplification product is detected with a hybridization probe, such as described above using the LightCycler FastStart DNA Master Hybridization Probes kit (Roche Molecular Biochemicals, Laval, Quebec, Canada) or alternative commercially available techniques such as ABI Taqman® technology (using for example an ABI Prism 7700 instrument to detect accumulation of PCR products continuously during the PCR process, Applied Biosystems, Foster City, California, U.S.A.). Altenative PCR methods and variations on the forgoing methods may be adopted, as for example are disclosed in the following U.S. Patents 6,180,349 (Ginzinger et al; Jan. 30, 2001); 6,033,854 (Kuit et al; March 7, 2000); 5,972,602 (Hyland; Oct. 26, 1999); 5,476,774 and 5,219,727 (Wang; Dec. 19, 1995 and June 15, 1993); 6,174,670 (Wittwer et al; Jan. 16, 2001); 6,143,496 (Brown; Nov. 7, 2000); 6,090,556 (Kato; July 18, 2000); 6,063,568 (Gerdes et al; May 16,2000).

### Plasma drug levels

The concentration of protease inhibitors (PIs) (indinavir, ritonavir, saquinavir, nelfinavir and lopinavir) and non-nucleoside reverse transcriptase inhibitors (NNRTIs) (nevirapine, delavirdine and efavirenz) were determined in the stored plasma samples that were collected for viral load testing. This was done using high performance liquid chromatography (HPLC) (HP 1100, Agilent Palo Alto, CA) coupled with tandem mass spectrometry (API-2000 LC/MS/MS System, AB/MDS-Sciex, Foster City, CA). Briefly, the PIs and NNRTIs were extracted with acetonitrile and precipitated plasma proteins were separated by filtration with Ultrafree-MC Filters (Millipore, Bedford, MA). The drugs in the filtrate were partially separated by HPLC on a Zorbax XDB C-18 column (Agilent Palo Alto, CA) and quantified by standard methods on the mass spectrometer. The samples were collected in acid citrate dextrose (ACD) tubes that dilute the blood somewhat and the time at which the last dose was administered was unknown. For these reasons, the plasma drug levels were considered to be a qualitative evaluation of whether the antiretrovirals were taken regularly and reaching the blood circulation.

### Statistical analysis

The Wilcoxon signed-rank test was used to assess paired differences between measurements (Table 3). Non-parametric Spearman's rho correlation coefficient was used to assess the correlation between clinical tests and the mtDNA/nDNA ratios.

### Results

The mitochondrial toxicity symptoms (N=8) were associated with markedly low mtDNA/nDNA ratios, 70% lower than HIV negative controls (N=24) and 45% lower than HIV positive/antiretroviral naive controls (N=47). The mtDNA ratios increased significantly following discontinuation of therapy (p=0.016). The decline in mtDNA preceded the increase in venous lactic acid levels and similarly, the post-therapy rebound in mtDNA appeared to precede a return to normal lactate levels. No significant correlation was observed between CD4 count (p=0.170) or platelet count (p=0.141) and the mtDNA/nDNA ratios.

### Patient characteristics

We retrospectively studied 8 HIV infected individuals enrolled in the Drug Treatment Program at the B.C. Centre for Excellence in HIV/AIDS at St.Paul's Hospital (Table 1). The patients experienced mitochondrial toxicity (MT) symptoms that included chronic hyperlactatemia, fatigue, rapid weight loss and low anaerobic threshold during cardiopulmonary exercise testing (data not shown). Two of the patients were on a drug regimen that consisted of 4 nucleoside analogues, 2 were taking hydroxyurea and all were receiving D4T at the time their MT symptoms developed. As a result of this drug toxicity, all had their antiretroviral therapy interrupted by their treating physician and their lactate levels monitored by RVLA over time. At the time of therapy interruption, 7/8 had a plasma viral load that was below the limit of detection of 50 copies/mL of plasma. After stopping antiretroviral therapy, MT symptoms gradually disappeared in all patients. The mean time off therapy was 15.6 weeks and 5/8 patients subsequently resumed antiretroviral therapy with a different drug regimen that excluded D4T and DDI, and achieved an undetectable plasma viral load. Two of the patients (4 and 5) had elevated liver enzymes prior to initiating their pre-interruption regimen. All others showed normal liver enzymes, INR and albumin levels before developing MT symptoms (data not shown).

### MtDNA /nDNA ratios and antiretroviral therapy

Longitudinal blood samples from 8 patients (between 6 and 17 distinct samples per patient, covering a period of 22 to 28 months) were collected before, during and after the antiretroviral therapy interruption and their mtDNA/nDNA ratios were determined (Figure 1c, 2; Table 2). As a control, mtDNA/nDNA ratios were determined for 24 healthy HIV negative males and 47 HIV positive drug naive males (Figure 1a,b; Table 2).

A statistical comparison of the ratios obtained with the various groups is presented in Table 3. The mean mtDNA/nDNA ratio of the HIV negative controls was significantly higher than that of the HIV positive/drug naive group. In the calculation of the pre-therapy interruption mean, all samples were considered, including those for which prescribed PI and NNRTI plasma drug levels were either undetectable or measured at >2 standard deviations below the average trough concentration (according to the drug manufacturer monograph) (Figure 2). Post-therapy interruption samples included all samples collected after therapy interruption with no limitations on time. Patient 1 was excluded from this analysis since there was no buffycoat available from the period off antiretrovirals. Five of the 8 patients eventually resumed antiretroviral therapy that excluded the nucleoside analogues D4T and DDI from the new regimens (Table 1). All samples collected during that time were included in the calculation of the off D4T mean ratio.

For the 8 MT patients together, the mean mtDNA/nDNA ratio observed before therapy interruption (but at least one month after initiation of their last drug regimen) was significantly lower than those obtained for either the HIV negative or the HIV positive/antiretroviral naive control groups (p<0.001). Both the mean ratios measured during complete therapy interruption and off D4T therapy (which include off all antiretrovirals as well as on antiretroviral regimen that excludes D4T) were very similar to the mean obtained for the HIV positive/drug naïve controls. In fact, the mean mtDNA/nDNA ratio of all the samples collected pre-therapy interruption was significantly lower than both the mean ratio off all antiretrovirals (p=0.016) and the mean ratio off D4T (p=0.008) (Table 3).

Several additional patient clinical test results were investigated to determine whether they showed a relationship with the mtDNA/nDNA ratio. No significant correlation between the mtDNA/nDNA ratio and CD4 count, both in the HIV positive/antiretroviral naive group (p=0.593) and in the MT patient group (p=0.170). Platelets contain a few mtDNA molecules per cell (Shuster et al., 1988) which may influence the mtDNA/nDNA ratio. Platelet data was not available for the control groups but for the MT group, there was no significant correlation between the ratio and platelet count (p=0.14). Similarly, for the MT patients, no correlation was found between the ratio and the white blood cell count (p=0.21), the alanine aminotransferase (ALT) (p=0.47), or the INR. However, a weak correlation was found between the mtDNA/nDNA ratio and the AST (p=0.02) as well as the albumin level (p=0.02).

### MtDNA and lactate levels

In patients 1, 4 and 8 (Figure 2) the mtDNA depletion clearly preceded the hyperlactatemia (earlier lactate data was unavailable for the other five patients). Similarly, in patients 4, 6 and 8, the time required for mtDNA levels to rebound was similar or shorter than that needed for the hyperlactatemia to normalize (0.5-2.1 mmol/L range). In several instances the mtDNA rebound preceded plasma viral load rebound (data not shown). Based on the limited mtDNA/nDNA data available, the maximum mtDNA half-life was estimated to range from 4.5 weeks (patient 3) to 8 weeks (patient 4) and the maximum mtDNA doubling times were estimated to range from 4 (patient 5) to 16 weeks (patient 1). Short lapses in therapy (as seen for patients 3, 4, 7) as well as extremely low circulating drug levels (patients 4, 6, 7) also affected mtDNA levels upward. Based on the data available, the maximum time off drugs before lactate levels returned to the normal range varied from 4 weeks (patient 8) to 28 weeks (patient 5). Furthermore, lactate levels remained within the normal range for months after resuming therapies that included 3TC + abacavir (ABA) or zidovudine (AZT). The difference between venous lactate levels on and off antiretroviral therapy did not reach significance (Table 3). This is most likely due to the lag time between changes in therapy and changes in lactate levels, a lag that was also present between changes in mtDNA/nDNA ratios and lactate levels. (see Figure 2, patients 1 and 4).

**Table 1. Characteristics of the eight patients with symptomatic mitochondrial toxicity and their antiretroviral therapy regimens.**

| Before stopping therapy | | | | | Off therapy | | After resuming therapy | |
|---|---|---|---|---|---|---|---|---|
| | Age/ | | Time | last | Time off | highest | | Time to HIV-1 pVL |
| patient | Gender | Drug Regimen^{c} | on D4T^{a} | HIV-1 pVL^{b} | Therapy | HIV-1 pVL | Drug Regimen | <50 copies/mL |
| | | | (weeks) | (copies/mL) | (weeks) | (copies/mL) | | (weeks) |
| 1 | 47/M | D4T/DDI/3TC/ABA/HU | 175 | <50 | 13 | 223.000 | SAQ/RIT/NEV | 12 |
| 2 | 41/M | D4T/DDI/3TC/SAQ/DEL/NEL/NEV/ABA/HU | 144 | <50 | 45+ | 178,000 | N/A | N/A |
| 3 | 44/M | D4T/DDI/3TC/ABA | 59 | 90 | 15 | 177,000 | 3TC/ABA/NEV/ABR | 18 |
| 4^{d} | 48/M | D4T/3TC/SAQ/RIT | 58 | <50 | 17 | 584,000 | 3TC/ABA/NEV/ABR | 20 |
| 5 | 41/M | D4T/DDI/EFV | 33 | <50 | 17 | 425,000 | 3TC/SAQ/RIT/EFV | 17 |
| 6 | 57/M | D4T/DDI/EFV | 33 | <50 | 17 | 750,010 | AZT/3TC/SAQ/RIT/EFV | 17 |
| 7 | 44/M | D4T/DDI/3TC/SAQ/IND/NEV/ABA/ABR | 192 | <50 | 28+ | 63,300 | N/A | N/A |
| 8 | 43/M | D4T/INDIDEL | 143 | <50 | 26+ | 138.000 | N/A | N/A |

| | | | | | | | | |
|---|---|---|---|---|---|---|---|---|
| a) Time the patient had continuously been prescribed D4T as part of their drug regimen, before stopping therapy. | | | | | | | | |
| b) Last plasma viral load before stopping antiretrovirals. | | | | | | | | |
| c) The abbreviations used are: D4T, stavudine; DDI, didanosine; 3TC, lamivudine; ABA, abacavir; SAQ, saquinavir; IND, indinavir; RIT, ritonavir; NEL, nelfinavir; ABR, lopinavir; DEL, delavirdine; NEL, nelfinavir; EFV, efavirenz; HU, hydroxyurea. | | | | | | | | |
| d) Patient co-infected with hepatitis C virus. | | | | | | | | |
| e) Table 2. Mitochondrial DNA/nuclear DNA ratios measured for the different groups. | | | | | | | | |

| HIV status/Antiretrovirals status | N^{a} (number of mtDNA/nDNA) | mtDNA/nDNA | |
|---|---|---|---|
| | | mean ± S.D. | (range) |
| HIV negative | 24 (24) | 1.283 ± 0.377 | (0.766-2.441) |
| HIV positive/Antiretroviral naïve | 47 (47) | 0.717 ± 0.189 | (0.368-1.098) |
| HIV positive with MT/on therapy | 8 (37) | 0.392 ± 0.143 | (0.184-0.856) |
| HIV positive with MT/off therapy | 8 (17) | 0.712 ± 0.203 | (0.394-1.228) |
| HIV positive with MT/off D4T | 8 (38) | 0.698 ± 0.171 | (0.394-1.228) |

| | | | |
|---|---|---|---|
| a) N = number of individuals within a given group, number of mtDNA/nDNA refers to the number of individual data points considered in the calculation of the mean value for that group. | | | |

On therapy data are those gathered while the patients were on their last regimen since ≥ 1 month.

Off therapy means off all antiretrovirals

Off D4T means off all antiretrovirals and/or on ARV therapy that does not include D4T.

**Table 3.**

| Comparison of mean values | data compared | P value^{a} |
|---|---|---|
| HIV negative vs 8 patients with MT/on ARV | mtDNA/nDNA ratio | <0.001 |
| HIV negative vs HIV positive/ARV naive | mtDNA/nDNA ratio | <0.001 |
| HIV positive/ARV naive vs 8 patients with MT/on ARV | mtDNA/nDNA ratio | <0.001 |
| 8 patients with MT/on ARV vs off all ARV | mtDNA/nDNA ratio | 0.016 |
| 8 patients with MT/on ARV vs off D4T | mtDNA/nDNA ratio | 0.008 |
| 8 patients with MT/on ARV vs off all ARV | lactate | 0.313 |
| 8 patients with MT/on ARV vs off D4T | lactate | 0.109 |

| | | |
|---|---|---|
| a) Determined by Wilcoxon signed-rank test. | | |

### Example 2

In one aspect, it has been found that assays of the invention may be used on post-mortem tissues to provide information relating to organ failure characterized by mitochondrial damage. In this example, post mortem analysis of tissues correlated well with cause of death. In a case where the cause of death was lactic acidosis and hepatic steatosis, mtDNA/nDNA ratios were reduced in liver compared to HIV+ and HIV- control samples. In a case where kidney failure was present at death, mtDNA/nDNA ratios were significantly reduced in kidney tissues compared to controls.

### References

Anderson CM, Clevenger W, Decker DK and Graho K. Modulation of mitochondrial Mass and Function for the treatment of diseases and for target and drug discovery. WO 01 /35096 filed by Mitokor on November 13, 2000 and published or May 17 2001.

Amaudo E, Dalakas M, Shanske S, Moraes CT, DiMauro S, Schon EA. Depletion of muscle mitochondrial DNA in AIDS patients with zidovudine-induced myopathy. *Lancet* 1991; **337**:508-510.

Bartley PB, Westacott L, Boots RJ, Lawson M, Potter JM, Hyland VJ, et al. Large hepatic mitochondrial DNA deletions associated with L-lactic acidosis and highly active antiretroviral therapy. *AIDS* 2001; **15**:419-420.

Berger A, Bruschek M, Grethen C, Sperl W, Kofler B. Poor storage and handling of tissue mimics mitochondrial DNA depletion. *Diagn Mol Pathol* 2001;**10**:55-59.

Brinkman K: Editorial response: Hyperlactatemia and hepatic steatosis as features of mitochondrial toxicity of nucleoside analogue reverse transcriptase inhibitors. *Clin lnfect Dis* 2000; **31**:167-169.

Brinkman K, Smeitink JA, Romijn JA, Reiss P. Mitochoridrial toxicity induced by nucleoside-analogue reverse transcriptase inhibitors is a key factor in the pathogenesis of antiretroviral-therapy-related lipodystrophy. *Lancet* 1999; **354**:1112-1114.

Brinkman K, Hadewynch JM, ter Hofstede HJM, Burger DM, Smeitink JAM, Koopmans PP. Adverse effects of reverse transcriptase inhibitors: mitochondrial toxicity as common pathway. *AIDS* 1998; **12**:1735-1744.

Chen C-H, Vazquez-Padua M, Cheng Y-C. Effect of anti-human immunodeficiency virus nucleoside analogues on mitochondrial DNA and its implication for delayed toxicity. *Mol Pharmacol* 1991; **39**:625-628.

Church YA, Mitchell WG Gonzalez - Gomez 1, Christensen J, Vu TH Dinauro S and Bolés RG. Mitochondrial DNA depletion, near-fatal metabolic acidosin, and liver failure in an HIV- infected child treated with combination antiretroviral therapy. J. Pediatries 2001 ; 138 (5): 748-751.

Fortgang IS, Belitsos PC, Chaisson RE, Moore RD. Hepatomegaly and steatosis in HIV-infected patients receiving nucleoside analog antiretroviral therapy, *Am J Gastroenterol* 1995; **90**:1433-1436.

Fouty B, Frerman F, Reeves R. Riboflavin to treat nucleoside analogue-induced lactic acidosis. *Lancet* 1988; **352**:291-292.

Gerard Y, Maulin L, Yazdanpanah Y, de la Tribonniere X, Amiel C, Maurage CA, et al. Symptomatic hyperlactatemia: an emerging complication of antiretioviral therapy. *AIDS* 2000*;***14***:*2723-2730.

Haas RHA. Comparison of genetic mitochondrial disease and nucleoside analogue toxicity. Ann NY Acad Sci 2000; **918**:247-261.

Harris M, Tesiorowski A, Chan K, Hogg R, Rosenberg F, Chan Yan C, et al. Lactic acidosis complicating antiretroviral therapy: frequency and correlates. *Antiviral Ther* 2000; **5**(Suppl 2):31 (abstract #34).

Harris M, Tesiorowski A, Thompson C, Kiess M, Rosenberg F, Chan Yan C, et al. *Antiviral Ther* 2000; **5**(Suppl 2):31 (abstract#35).

John M, Moore CB, James IR, Nolan D, Upton RP, MeKinnon EJ, et al. Chronic hyperlactatemia in HIV-infected patients taking antiretroviral therapy. *AIDS* 2001; **15**:717-723.

Kakuda TN. Pharmacology of nucleoside and nucleotide reverse transcriptase inhibitor-induced mitochondrial toxicity. *Clin Ther* 2000; **22**(6):685-708.

Kakuda TN, Brundage RC, Anderson PL, Fletcher CV. Nucleoside reverse transcriptase inhibitor-induced mitochondrial toxicity as an etiology for lipodystrophy. (1999) AIDS 1999; **13**:2311-2312.

Lewis W, Dalakas MC. Mitochondrial toxicity of antiviral drugs. *Nature Med* 1995; **1**:417-422.

Lewis W, Gonzalez B, Chomyn A, Papoian T. Zidovudine induces molecular, biochemical, and ultrastructural changes in rat skeletal muscle mitochondria. *J Clin Invest* 1992; **89**:1354-1360.

Lonergan JT, Behling C, Pfander H, Hassanein TI Mathews WC. Hyperlacatatemia and hepatic abnormalities in 10 human immunodeficiency virus-infected patients receiving nucleoside analogue combination regimens. Clin Infect Dis 2000; **31**:162-166.

Martin JL, Brown CE, Matthews-Davis N Reardon JE. Effects of antiviral nucleoside analogs on human DNA polymerases and mitochondrial DNA synthesis. *Antimicrob Agents Chentother* 1994; **38**:2743-2749.

Medina DJ, Tsai C-H, Hsiung GD, Cheng Y-C. Comparison of mitochondrial morphology, mitochondrial DNA content, and cell viability in cultured cells treated with three anti-human immunodeficiency virus dideoxynucleosides. *Antimicrob Agents Chemother* 1994; **38**:1824-1828.

Megarbane B, Brivet F, Guérin JM, Baud FJ. Acidose lactique et défaillance multi-viscérale secondaire aux thérapeutiques antirétrovirales chez les patients infectés par le VIH. *La Presse Med* 2000; **40**:2257-2264.

Moraes CT, Kenyon L, Hao H. Mechanisms of human mitochondrial DNA maintenance: The determining role of primary sequence and length over function. *Mol Biol Cell* 1999, **10**:3345-3356.

Moyle G. Clinical manifestation and management of antiretroviral nucleoside analog-related mitochondrial toxicity. *Clin Ther* 2000; **22**:911-936.

Pan-Zhou X-R, Cui L, Zhou X-J, Sommadossi J-P, Darley-Usmar VM. Differential effects of antiretroviral nucleoside analogs on mitochondrial function in HepG2 cells. *Antimicrob Agents Chemother* 2000; **44**:496-503.

Shadel GS, Clayton DA. Mitochondrial DNA maintenance in vertebrates. *Ann Rev Biochem* 1997; **66**:409-435.

Shuster RC, Rubenstein AJ, Wallace DC. Mitochondrial DNA in anucleate human blood cells. *Biochem Biophys Res Commun* 1988; **155**:1360-1365.

Tang Y, Schon EA, Wilichowski E, Vazquez-Memije ME, Davidson E, King MP. Rearrangements of human mitochondrial DNA (mtDNA): New insight into the regulation of mtDNA copy number and gene expression. *Mol Biol Cell* 2000; **11**:1471-1485.

Wallace DC. Mitochondrial disease in man and mouse. *Science* 1999; **283**:1482-1488.

Zheng H, Cooney DA, Sreenath A, Zhan Q, Agbaria R, Stowe EE, Fornace AJ and Johns DG. Quantitation of mitochondrial DNA in human lymphoblasts by a competitive polymerase chain reaction method: application to the study of inhibitors of mitochondrial DNA content. *Mol Pharmacol 1994;* 46(6): 1063-1069.

### Conclusion

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way. Numeric ranges are inclusive of the numbers defining the range. In the specification, the word "comprising" is used as an open-ended term, substantially equivalent to the phrase "including, but not limited to", and the word "comprises" has a corresponding meaning. Citation of references herein shall not be construed as an admission that such references are prior art to the present invention. The invention includes all embodiments and variations substantially as hereinbefore described and with reference to the examples and drawings.

## Claims

1. An in vitro method for monitoring toxicity of a nucleic acid precursor drug treatment, comprising measuring the mitochondrial DNA content relative to the amount of nuclear DNA in cells in a peripheral blood sample from a subject undergoing treatment with the drug.

2. The method of claim 1, further comprising the step of determining whether the ratio of mitochondrial DNA to nuclear DNA falls below a predetermined level of 0.5 of a baseline ratio, wherein the baseline ratio is measured before the subject is treated with the drug, or is measured in a control subject.

3. The method of claim 2, wherein the predetermined level is 0.45 of the baseline ratio.

4. The method of claim 2, wherein the predetermined level is 0.40 of the baseline ratio.

5. The method of claim 2, wherein the predetermined level is 0.35 of the baseline ratio.

6. The method of claim 2, wherein the predetermined level is 0.30 of the baseline ratio.

7. The method of any one of claims I to 6, wherein the amount of DNA is measured by a polymerase chain reaction.

8. The method of claim 7, wherein the polymerase chain reaction is a quantitative polymerase chain reaction, wherein amplification of the mitochondrial DNA is compared to amplification of a reference DNA.

9. The method of claim 7, wherein the polymerase chain reaction is a real-time polymerase chain reaction wherein an amplification product is detected with a hybridization probe.

10. The method of any one of claims 1 through 9, wherein the drug is a nucleoside or nucleotide analogue.

11. The method of claim 10, wherein the nucleoside analogue is selected from the group consisting of AZT, ddI, ddC, d4T, 3Tc, Abacavir and D4T.

12. The method of claim 10, wherein the nucleoside analogue is D4T.

13. The method of claim 10, wherein the nucleotide analogue is selected from the group consisting of Tenofovir and Cidofovir.

14. The method of any one of claims 1 through 9, wherein the drug is a reverse transcriptase inhibitor.

15. The method of any one of claims 1 through 14, wherein the subject is a human patient.

16. The method of any one of claims 1 through 14, wherein the subject is a non-human animal.

17. The method of claim 15, wherein the subject is suffering from an HIV infection.

18. The method of any one of claims 1 through 17, wherein the consequence of said toxicity is chosen from the group consisting of myopathy, cardiomyopathy, neuropathy, liver steatosis, nephrotic toxicity, pancreatitis, decreased oxidative phosphorylation, lactic acidosis, lipid abnormalities, hyperlactatemia, low anaerobic threshold in cardiopulmonary testing, fatigue and rapid weight loss.

## Patentansprüche

1. In vitro-Verfahren für die Überwachung der Toxizität einer Behandlung mit einem Nukleinsäurevorläufer-Wirkstoff, wobei das Verfahren das Messen des mitochondrialen DNA-Gehalts im Verhältnis zu der Menge nukleärer DNA in Zellen in einer Probe peripheren Bluts von einem Subjekt umfasst, das sich der Behandlung mit dem Wirkstoff unterzieht.

2. Verfahren gemäß Anspruch 1, weiter umfassend den Schritt, zu bestimmen, ob das Verhältnis von mitochondrialer DNA zu nukleärer DNA unter ein vorbestimmtes Niveau von 0,5 eines Basisverhältnisses fällt, wobei das Basisverhältnis gemessen wird, bevor das Subjekt mit dem Wirkstoff behandelt wird, oder wobei es in einem Kontrollsubjekt gemessen wird.

3. Verfahren gemäß Anspruch 2, wobei das vorbestimmte Niveau 0,45 des Basisverhältnisses ist.

4. Verfahren gemäß Anspruch 2, wobei das vorbestimmte Niveau 0,40 des Basisverhältnisses ist.

5. Verfahren gemäß Anspruch 2, wobei das vorbestimmte Niveau 0,35 des Basisverhältnisses ist.

6. Verfahren gemäß Anspruch 2, wobei das vorbestimmte Niveau 0,30 des Basisverhältnisses ist.

7. Verfahren gemäß einem der Ansprüche 1 bis 6, wobei der Gehalt an DNA mittels einer Polymerase-Kettenreaktion gemessen wird.

8. Verfahren gemäß Anspruch 7, wobei die Polymerase-Kettenreaktion eine quantitative Polymerase-Kettenreaktion ist, wobei die Amplifizierung der mitochondrialen DNA mit der Amplifizierung einer Referenz-DNA verglichen wird.

9. Verfahren gemäß Anspruch 7, wobei die Polymerase-Kettenreaktion eine Echtzeit-Polymerase-Kettenreaktion ist, wobei ein Amplifikationsprodukt mittels einer Hybridisierungssonde nachgewiesen wird.

10. Verfahren gemäß einem der Ansprüche 1 bis 9, wobei der Wirkstoff ein Nukleosid- oder Nukleotid-Analogon ist.

11. Verfahren gemäß Anspruch 10, wobei das Nukleosid-Analogon aus der Gruppe, die aus AZT, ddI, ddC, d4T, 3Tc, Abacavir und D4T besteht, ausgewählt wird.

12. Verfahren gemäß Anspruch 10, wobei das Nukleosid-Analogon D4T ist.

13. Verfahren gemäß Anspruch 10, wobei das Nukleotid-Analogon aus der Gruppe, die aus Tenofovir und Cidofovir besteht, ausgewählt wird.

14. Verfahren gemäß einem der Ansprüche 1 bis 9, wobei der Wirkstoff ein Inhibitor der reversen Transkriptase ist.

15. Verfahren gemäß einem der Ansprüche 1 bis 14, wobei das Subjekt ein humaner Patient ist.

16. Verfahren gemäß einem der Ansprüche 1 bis 14, wobei das Subjekt ein nicht-humanes Tier ist.

17. Verfahren gemäß Anspruch 15, wobei das Subjekt an einer HIV-Infektion leidet.

18. Verfahren gemäß einem der Ansprüche 1 bis 17, wobei die Folge der Toxizität aus der Gruppe, die aus Myopathie, Kardiomyopathie, Neuropathie, Nierentoxizität, Pankreatitis, verminderter oxidativer Phosphorylierung, Lactatazidose, Lipid-Anomalien, Lebersteatose, Hyperlactatämie, niedrigem anaeroben Schwellenwert bei kardiopulmonaler Überprüfung, Erschöpfung und schnellem Gewichtsverlust besteht, ausgewählt wird.

## Revendications

1. Procédé in vitro de surveillance de la toxicité dans un traitement par médicament précurseur à base d'acide nucléique, comprenant la mesure de la teneur en ADN mitochondrial par rapport à la quantité d'ADN nucléaire dans les cellules dans un échantillon de sang périphérique provenant d'un sujet subissant un traitement avec le médicament.

2. Procédé selon la revendication 1, comprenant en outre l'étape consistant à déterminer si le rapport de l'ADN mitochondrial à l'ADN nucléaire tombe au-dessous d'un niveau prédéterminé de 0,5 d'un rapport de base, dans lequel le rapport de base est mesuré avant que le sujet ne soit traité avec le médicament, ou est mesuré chez un sujet témoin.

3. Procédé selon la revendication 2, dans lequel le niveau prédéterminé est de 0,45 du rapport de base.

4. Procédé selon la revendication 2, dans lequel le niveau prédéterminé est de 0,40 du rapport de base.

5. Procédé selon la revendication 2, dans lequel le niveau prédéterminé est de 0,35 du rapport de base.

6. Procédé selon la revendication 2, dans lequel le niveau prédéterminé est de 0,30 du rapport de base.

7. Procédé selon l'une quelconque des revendications 1 à 6, dans lequel la quantité d'ADN est mesurée par une amplification en chaîne par polymérase.

8. Procédé selon la revendication 7, dans lequel l'amplification en chaîne par polymérase est une amplification en chaîne par polymérase quantitative, dans lequel l'amplification de l'ADN mitochondrial est comparée à l'amplification d'un ADN de référence.

9. Procédé selon la revendication 7, dans lequel l'amplification en chaîne par polymérase est une amplification en chaîne par polymérase en temps réel, dans lequel un produit d'amplification est détecté avec une sonde d'hybridation.

10. Procédé selon l'une quelconque des revendications 1 à 9, dans lequel le médicament est un analogue de nucléoside ou de nucléotide.

11. Procédé selon la revendication 10, dans lequel l'analogue de nucléoside est choisi dans le groupe constitué par AZT, ddI, ddC, d4T, 3Tc, Abacavir et D4T.

12. Procédé selon la revendication 10, dans lequel l'analogue de nucléoside est D4T.

13. Procédé selon la revendication 10, dans lequel l'analogue de nucléotide est choisi dans le groupe constitué par le Ténofovir et le Cidofovir.

14. Procédé selon l'une quelconque des revendications 1 à 9, dans lequel le médicament est un inhibiteur de la transcriptase inverse.

15. Procédé selon l'une quelconque des revendications 1 à 14, dans lequel le sujet est un patient humain.

16. Procédé selon l'une quelconque des revendications 1 à 14, dans lequel le sujet est un animal non humain.

17. Procédé selon la revendication 15, dans lequel le sujet souffre d'une infection par VIH.

18. Procédé selon l'une quelconque des revendications 1 à 17, dans lequel la conséquence de ladite toxicité est choisie dans le groupe constitué par une myopathie, une cardiomyopathie, une neuropathie, une stéatose du foie, une toxicité néphrotique, une pancréatite, une phosphorylation oxydative réduite, une acidose lactique, des anomalies lipidiques, une hyper lactatémie, un seuil d'anaérobiose faible dans un test cardiorespiratoire, une fatigue et une perte de poids rapide.


### SEQUENCE LISTING

<110> THE UNIVERSITY OF BRITISH COLUMBIA
   COTE, Helene
   MONTANER, Julio
   O'SHAUGHNESSY, Michael
<120> PHARMACOLOGICAL APPLICATIONS OF MITOCHONDRIAL DNA ASSAYS
<130> 80021-367
<140> PCT/CA02/00796
   <141> 2002-05-29
<150> US 60/293,523
   <151> 2001-05-29
<160> 8
<170> PatentIn version 3.1
<210> 1
   <211> 20
   <212> DNA
   <213> Artificial Sequence
<220>
   <223> Primer
<400> 1
   ttcgccgacc gttgactatt 20
<210> 2
   <211> 23
   <212> DNA
   <213> Artificial Sequence
<220>
   <223> Primer
<400> 2
   aagattatta caaatgcatg ggc 23
<210> 3
   <211> 22
   <212> DNA
   <213> Artificial Sequence
<220>
   <223> Primer
<220>
   <221> misc_feature
   <222> (22)..(22)
   <223> 3' Fluorescein-labeled
<400> 3
   gccagccagg caaccttcta gg 22
<210> 4
   <211> 29
   <212> DNA
   <213> Artificial Sequence
<220>
   <223> Primer
<220>
   <221> misc_feature
   <222> (1)..(1)
   <223> 5'LC Red640-labeled
<220>
   <221> misc_feature
   <222> (29)..(29)
   <223> 3' end Phosphate-blocked
<400> 4
   aacgaccaca tctacaacgt tatcgtcad 29
<210> 5
   <211> 20
   <212> DNA
   <213> Artificial Sequence
<220>
   <223> Primer
<400> 5
   gagctgttga cggaaaggag 20
<210> 6
   <211> 21
   <212> DNA
   <213> Artificial Sequence
<220>
   <223> Primer
<400> 6
   cagaagagaa tcccggctaa g 21
<210> 7
   <211> 27
   <212> DNA
   <213> Artificial Sequence
<220>
   <223> Primer
<220>
   <221> misc_feature
   <222> (27)..(27)
   <223> 3' Fluorescein-labeled
<400> 7
   gaggcgctgt tagagatctg tcagaga 27
<210> 8
   <211> 25
   <212> DNA
   <213> Artificial Sequence
<220>
   <223> Primer
<220>
   <221> misc_feature
   <222> (1).. (1)
   <223> 5'LC Red640-labeled
<220>
   <221> misc feature
   <222> (25) .. (25)
   <223> 3' end Phosphate-blocked
<400> 8
   ggcatttcct aagtggaagc aagca 25